(19) Europäisches Patentamt
European Patent Office
Office européen des brevets

(11) Veröffentlichungsnummer: **0 307 753 B1**

(12) # EUROPÄISCHE PATENTSCHRIFT

(45) Veröffentlichungstag der Patentschrift: **11.11.92**

(51) Int. Cl.[5]: **C07D 251/16**, A01N 47/36, A01N 47/32, //C07C311/00

(21) Anmeldenummer: **88114480.2**

(22) Anmeldetag: **05.09.88**

Die Akte enthält technische Angaben, die nach dem Eingang der Anmeldung eingereicht wurden und die nicht in dieser Patentschrift enthalten sind.

(54) **Sulfonyliso(thio)harnstoffe sowie ein Verfahren und neue Zwischenprodukte zu ihrer Herstellung.**

(30) Priorität: **16.09.87 DE 3731053**

(43) Veröffentlichungstag der Anmeldung:
**22.03.89 Patentblatt 89/12**

(45) Bekanntmachung des Hinweises auf die Patenterteilung:
**11.11.92 Patentblatt 92/46**

(84) Benannte Vertragsstaaten:
**BE CH DE ES FR GB IT LI NL SE**

(56) Entgegenhaltungen:
**EP-A- 0 005 986** **EP-A- 0 030 433**
**EP-A- 0 107 624** **EP-A- 0 112 289**
**DE-A- 3 517 844** **DE-A- 3 634 927**

**CHEMICAL ABSTRACTS, Band 98, Nr. 9, 28. Februar 1983, Columbus, Ohio, US; MERCHAN, F.L. et al.: "Synthesis of 2-sulfonylaminobenzimidazoles and 4,5-dicyano-2-sulfonylaminoimidazoles from N-dichloromethylenesulfonamides" Seite 623, Spalte 2, Zusammenfassung-Nr. 72 002y**

(73) Patentinhaber: **BAYER AG**

**W-5090 Leverkusen 1 Bayerwerk(DE)**

(72) Erfinder: **Riebel, Hans-Jochem, Dr.**
**In der Beek 92**
**W-5600 Wuppertal 1(DE)**
Erfinder: **Fest, Christa, Dr.**
**Im Johannistal 20**
**W-5600 Wuppertal 1(DE)**
Erfinder: **Müller, Klaus-Helmut, Dr.**
**Bockhackstrasse 55**
**W-4000 Düsseldorf 13(DE)**
Erfinder: **Santel, Hans-Joachim, Dr.**
**Gruenstrasse 9 a**
**W-5090 Leverkusen 1(DE)**
Erfinder: **Schmidt, Robert R., Dr.**
**Im Waldwinkel 110**
**W-5060 Bergisch Gladbach 2(DE)**
Erfinder: **Strang, Harry, Dr.**
**Unterdorfstrasse 6 a**
**W-4000 Düsseldorf 31(DE)**

**EP 0 307 753 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 67, Nr. 21, 20. November 1967, Columbus, Ohio, US; E.KUEHLE et al.: "Synthesis of dihalo isocyanides" Seite 9378, Spalte 1, Zusammenfassung-Nr. 99 821m

CHEMICAL ABSTRACTS, Band 78, Nr. 17, 30. April 1973, Columbus, Ohio, US; GORDIENKO, N.S. et al.: "Tautomerism and acid properties of acyl derivatives of benzenesulfonamides in alcohols, ketones, and ethers" Seite 412, Spalte 1, Zusammenfassung-Nr. 110 398d

CHEMICAL ABSTRACTS, Band 77, Nr. 9, 28. August 1972, Columbus, Ohio, US; MARKOV, V.I. et al.: "Alkylation of disodium salts of arylsulfimidodithiocarbonic acid" Seite 457, Spalte 2, Zusammenfassung-Nr. 61 443h

CHEMICAL ABSTRACTS, Band 83, Nr. 21, 24. November 1975, Columbus, Ohio, US; DUBINA, V.L. et al.: "Arylsulfonylbenzimidoyl chlorides. IX. Synthesis of mixed anhydrides of N-(arylsulfonyl)imidobenzoic and certain carboxylic acids" Seite 523, Spalte 1, Zusammenfassung-Nr. 178 491n

## Beschreibung

Die Erfindung betrifft neue Sulfonyliso(thio)harnstoffe sowie ein erfinderisches Verfahren und neue Zwischenprodukte zu ihrer Herstellung und ihre Verwendung als Herbizide.

Es ist bekannt, daß bestimmte Sulfonylisoharnstoffe, wie z. B. N′-(2-Methoxycarbonyl-phenylsulfonyl)-N″-(4,6-dimethoxy-pyrimidin-2-yl)-O-phenylisoharnstoff, herbizide Eigenschaften aufweisen (vgl. EP-A 173 957).

Die Wirkung dieser Verbindungen sowie der Verlauf des bekannten Verfahrens zu ihrer Herstellung über entsprechende Sulfonylguanidine sind jedoch nicht in allen Fällen ganz zufriedenstellend.

Es wurden nun neue Sulfonyliso(thio)harnstoffe der allgemeinen Formel (I)

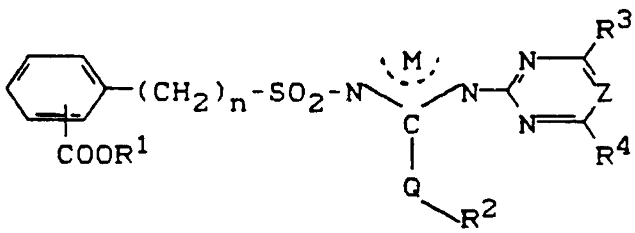

(I)

in welcher

- M für Wasserstoff oder ein Alkalimetall- oder Erdalkalimetall-äquivalent steht,
- n für die Zahlen Null oder 1 steht,
- Q für Sauerstoff oder Schwefel steht,
- $R^1$ für gegebenenfalls durch Halogen oder $C_1$-$C_4$-Alkoxy substituiertes $C_1$-$C_6$-Alkyl steht,
- $R^2$ für einen Phenylrest steht, welcher gegebenenfalls substituiert ist durch einen oder mehrere Reste aus der Reihe
  Halogen [wie insbesondere Fluor, Chlor, Brom und Iod], Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1$-$C_4$-Alkoxy-carbonylamino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist;
- $R^3$ für gegebenenfalls durch Halogen substituiertes $C_1$-$C_2$-Alkyl oder $C_1$-$C_2$-Alkoxy steht,
- $R^4$ für gegebenenfalls durch Halogen substituiertes $C_1$-$C_2$-Alkyl oder $C_1$-$C_2$-Alkoxy steht und
- Z für N steht,

gefunden.

Weiter wurde ein erfinderisches Verfahren zur Herstellung der neuen Sulfonyliso(thio)harnstoffe der allgemeinen Formel (I) gefunden, welches dadurch gekennzeichnet ist, daß man N-Sulfonyl-imino-(dithio)-kohlensäure-diester der allgemeinen Formel (II)

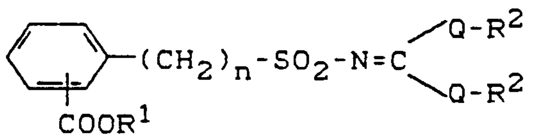

(II)

in welcher

n, Q, $R^1$ und $R^2$ die oben angegebenen Bedeutungen haben,

mit Aminoverbindungen der allgemeinen Formel (III)

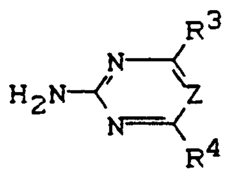

(III)

in welcher

$R^3$, $R^4$ und Z die oben angegebenen Bedeutungen haben,

oder mit Alkalimetall- bzw. Erdalkalimetall-salzen hiervon in Gegenwart eines Verdünnungsmittels und gegebenenfalls in Gegenwart einer basischen Metallverbindung bei Temperaturen zwischen 0 °C und 100 °C umsetzt.

Überraschenderweise können nach dem erfindungsgemäßen Herstellungsverfahren die Verbindungen der Formel (I) in hohen Ausbeuten und in guter Qualität erhalten werden. Es war nämlich damit zu rechnen, daß die in den Ausgangsstoffen der Formel (II) und in den Produkten der Formel (I) vorhandene Carbonsäureester-Gruppierung mit den Aminoverbindungen der Formel (III) unerwünschte Reaktionen eingehen würden.

Die neuen Sulfonyliso(thio)harnstoffe der allgemeinen Formel (I) zeichnen sich durch starke herbizide Wirksamkeit aus.

Überraschenderweise zeigen die neuen Vebindungen der Formel (I) erheblich bessere herbizide Wirkung als vorbekannte Sulfonylisoharnstoffe gleicher Wirkungsrichtung.

Gegenstand der Erfindung sind vorzugsweise Verbindungen der Formel (I), in welcher

M für Wasserstoff oder ein Natrium-, Kalium-, Magnesium- oder Calcium-äquivalent steht,

n für die Zahlen Null oder 1 steht,

Q für Sauerstoff oder Schwefel steht,

$R^1$ für Methyl, Ethyl, Propyl, Isopropyl, Butyl, Isobutyl, sec-Butyl, 2-Fluor-ethyl, 2-Chlor-ethyl, 2-Methoxy-ethyl oder 2-Ethoxyethyl steht,

$R^2$ für einen Phenylrest steht, welcher gegebenenfalls substituiert ist durch einen oder zwei Reste aus der Reihe

Fluor, Chlor, Brom, Iod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pydridoxy [welches gegebenenfalls durch Chlor und/oder Trifluormethyl substituiert ist], oder welcher gegebenenfalls benzanelliert ist;

$R^3$ für Methyl, Ethyl, Trifluormethyl, Methoxy, Ethoxy, Difluormethoxy oder Trifluorethoxy steht,

$R^4$ für Methyl, Methoxy, Ethoxy, Difluormethoxy oder Trifluorethoxy steht und

Z für N steht.

Gegenstand der Erfindung sind insbesondere Verbindungen der Formel (I), in welcher

M für Wasserstoff steht,

n für Null steht,

Q für Schwefel steht,

$R^1$ für Methyl oder Ethyl steht,

$R^2$ für gegebenenfalls durch Fluor, Chlor, Methyl oder Methoxy substituiertes Phenyl steht,

$R^3$ für Methyl oder Methoxy steht,

$R^4$ für Methoxy steht und

Z für N steht

- mit der Maßgabe, daß sich die Gruppierung $COOR^1$ in der ortho-Position befindet.

Verwendet man beispielsweise N-(2-Methoxycarbonyl-phenylsulfonyl)-imino-kohlensäure-diphenylester und das Kaliumsalz von 2-Amino-4,6-dimethoxy-s-triazin als Ausgangsstoffe, so kann der Reaktionsablauf beim erfindungsgemäßen Verfahren durch das folgende Formelschema wiedergegeben werden:

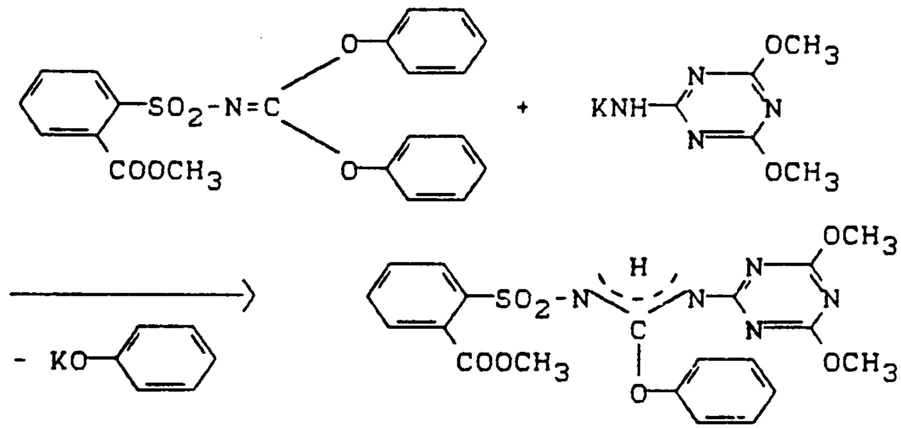

Die beim erfindungsgemäßen Verfahren als Ausgangsstoffe zu verwendenden N-Sulfonyl-imino-(dithio)-kohlensäure-diester sind durch die Formel (II) allgemein definiert. In Formel (II) haben n, Q, $R^1$ und $R^2$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Ausgangsstoffe der Formel (II) sind in der nachstehenden Tabelle 1 aufgeführt.

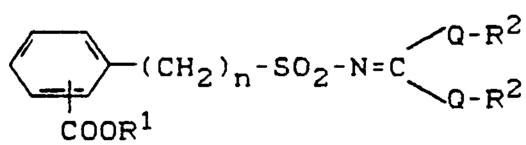

(II)

<u>Tabelle 1</u>: Beispiele für die Ausgangsstoffe der Formel (II)

| n | Q | $R^1$ | $R^2$ | Position $COOR^1$ |
|---|---|---|---|---|
| 0 | O | $CH_3$ | 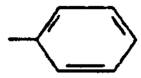 | ortho |
| 0 | O | $C_2H_5$ | 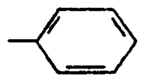 | ortho |
| 0 | S | $CH_3$ | 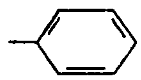 | ortho |
| 0 | S | $C_2H_5$ | 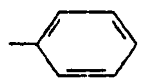 | ortho |
| 1 | O | $CH_3$ | 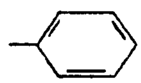 | ortho |
| 1 | O | $C_2H_5$ | 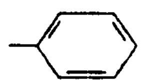 | ortho |
| 1 | S | $CH_3$ | 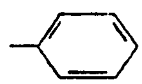 | ortho |
| 1 | S | $C_2H_5$ | 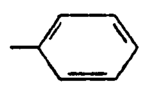 | ortho |
| 0 | O | $CH_3$ | 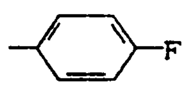 | ortho |
| 0 | O | $CH_3$ | 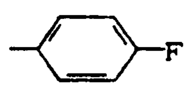 | ortho |

Tabelle 1 - Fortsetzung

| n | Q | $R^1$ | $R^2$ | Position $COOR^1$ |
|---|---|---|---|---|
| 0 | O | $C_2H_5$ | 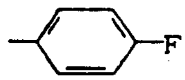 | ortho |
| 0 | S | $C_2H_5$ | 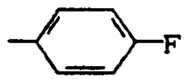 | ortho |
| 0 | O | $CH_3$ | 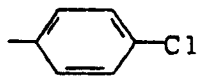 | ortho |
| 0 | S | $CH_3$ | 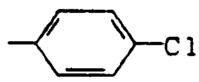 | ortho |
| 0 | O | $CH_3$ | 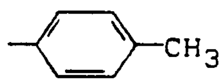 | ortho |
| 0 | S | $CH_3$ | 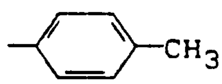 | ortho |
| 0 | O | $CH_3$ | 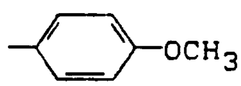 | ortho |
| 0 | S | $CH_3$ | 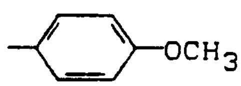 | ortho |
| 0 | O | $CH_3$ | 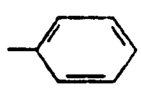 | meta |
| 0 | O | $CH_3$ | 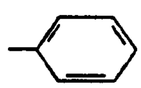 | para |

Tabelle 1 - Fortsetzung

| n | Q | $R^1$ | $R^2$ | Position $COOR^1$ |
|---|---|---|---|---|
| 0 | O | $C_2H_5$ | 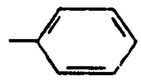 | para |
| 0 | S | $CH_3$ | 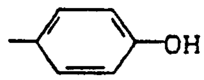 | ortho |
| 1 | S | $CH_3$ | 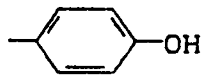 | ortho |
| 0 | O | $CH_3$ | 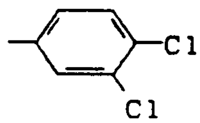 | ortho |
| 0 | S | $CH_3$ | 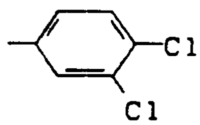 | ortho |
| 0 | O | $CH_3$ | 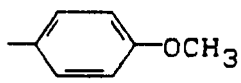 | para |
| 0 | S | $C_2H_5$ | 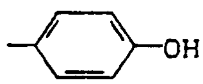 | ortho |
| 0 | O | $CH_3$ | 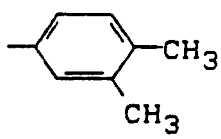 | ortho |
| 0 | O | $CH_3$ | 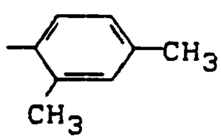 | ortho |

Tabelle 1 - Fortsetzung

| n | Q | $R^1$ | $R^2$ | Position $COOR^1$ |
|---|---|---|---|---|
| 0 | O | $C_2H_5$ | 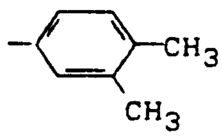 | ortho |
| 0 | O | $C_2H_5$ | 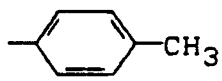 | ortho |
| 0 | S | $C_2H_5$ | 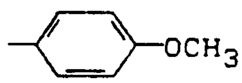 | ortho |

Die N-Sulfonyl-imino-(dithio)kohlensäure-diester der Formel (II) sind bisher nicht aus der Literatur bekannt. Man erhält die Verbindungen der Formel (II), wenn man Sulfonyl-isocyanid-dichloride der allgemeinen Formel (IV)

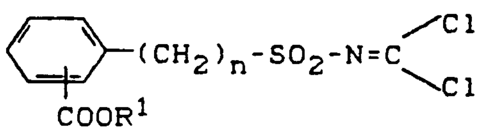 (IV)

in welcher

n und $R^1$ die oben angegebenen Bedeutungen haben,

mit Verbindungen der Formel (V)

$M - Q - R^2$ (V)

in welcher

M, Q und $R^2$ die oben angegebenen Bedeutungen haben,

in Gegenwart eines Verdünnungsmittels, wie z. B. Toluol, und gegebenenfalls in Gegenwart eines Säureakzeptors, wie z. B. Triethylamin, bei Temperaturen zwischen 0 °C und 50 °C umsetzt und nach üblichen Methoden aufarbeitet.

Bei den Ausgangsstoffen der Formel (V) haben M, Q und $R^2$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (V) seien genannt:

Phenol, Thiophenol, 4-Fluor-phenol und -thiophenol, 4-Chlor-phenol und -thiophenol, 4-Methyl-phenol und -thiophenol, 4-Hydroxy-phenol und -thiophenol, 4-Methoxy-phenol und -thiophenol, 3,4-Dimethyl-phenol und -thiophenol.

Die Verbindungen der Formel (V) sind bekannte Synthesechemikalien.

Bei den Ausgangsstoffen der Formel (IV) haben n und $R^1$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Ausgangsstoffe der Formel (IV) sind in der nachstehenden Tabelle 2 aufgeführt.

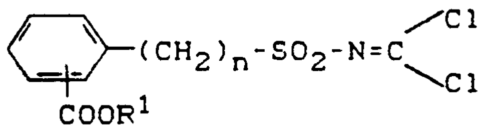 (IV)

Tabelle 2: Beispiele für die Ausgangsstoffe der Formel (IV)

| n | $R^1$ | Position $COOR^1$ |
|---|---|---|
| 0 | $CH_3$ | ortho |
| 1 | $CH_3$ | ortho |
| 0 | $C_2H_5$ | ortho |
| 1 | $C_2H_5$ | ortho |
| 0 | $CH_3$ | meta |
| 0 | $CH_3$ | para |
| 0 | $C_2H_5$ | para |

Die Verbindungen der Formel (IV) sind bisher nicht aus der Literatur bekannt. Man erhält die Sulfonyl-isocyanid-dichloride der Formel (IV), wenn man N-Sulfonyl-imino-dithiokohlensäure-dimethylesterder Formel (VI)

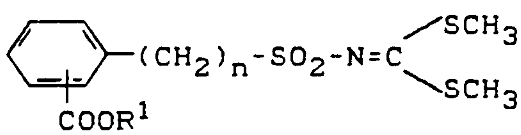 (VI)

in welcher

n und R die oben angegebenen Bedeutungen haben,

mit Chlor oder Sulfurylchlorid, gegebenenfalls in Gegenwart eines Verdünnungsmittels, wie z. B. Methylenchlorid, Chloroform oder Tetrachlormethan, bei Temperaturen zwischen 30 °C und 70 °C umsetzt und anschließend destilliert.

Bei den Ausgangstoffen der Formel (VI) haben n und $R^1$ vorzugsweise bzw. insbesondere die gleichen Bedeutungen, wie sie oben im Rahmen der Substituentendefinition der Formel (I) vorzugsweise bzw. als insbesondere bevorzugt angegeben sind.
Beispiele für die Ausgangsstoffe der Formel (VI) sind in der nachstehenden Tabelle 3 aufgeführt.

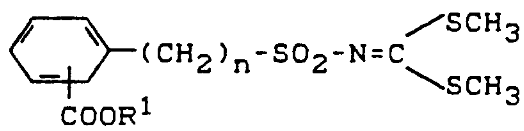

(VI)

<u>Tabelle 3</u>: Beispiele für die Aussgangsstoffe der Formel (VI)

| n | $R^1$ | Position $COOR^1$ |
|---|---|---|
| 0 | $CH_3$ | ortho |
| 1 | $CH_3$ | ortho |
| 0 | $C_2H_5$ | ortho |
| 1 | $C_2H_5$ | ortho |
| 0 | $CH_3$ | meta |
| 0 | $C_2H_5$ | meta |
| 0 | $CH_3$ | para |
| 0 | $C_2H_5$ | para |

Die N-Sulfonyl-imino-dithiokohlensäure-dimethylester der Formel (VI) sind - mit dem Synonym Isodithiocarbamidsäure-Derivate, wie z. B. N-(2-Methoxycarbonyl-benzolsulfonyl)-und N-(2-Ethoxycarbonyl-benzolsulfonyl-S,S-dimethyl-isodithiocarbamidsäureester - bekannt und/oder können nach an sich bekannten Verfahren hergestellt werden (vgl. EP-A 121 082).

Man erhält die N-Sulfonyl-imino-dithiokohlensäure-dimethylester der Formel (VI) auch nach einem erfinderischen Verfahren, welches Gegenstand einer nicht vorveröffentlichten Patentanmeldung ist, indem man Imino-dithiokohlensäure-dimethylester oder dessen Hydrochlorid mit Sulfonsäurehalogeniden der Formel (VII)

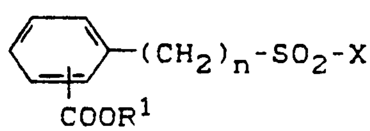

(VII)

in welcher

n und $R^1$ die oben angegebenen Bedeutungen haben und

X für Halogen, vorzugsweise für Chlor steht,

in Gegenwart eines Säureakzeptors, wie z. B. 1,4-Diazabicyclo-[2,2,2]-octan (DABCO), und in Gegenwart eines Verdünnungsmittels, wie z. B. Methylenchlorid, bei Temperaturen zwischen -20 °C und +50 °C umsetzt und nach üblichen Methoden aufarbeitet.

Imino-dithiokohlensäure-dimethylester und die Sulfonsäurehalogenide der Formel (VII) sind bekannt (vgl. DE-AS 12 52 656 bzw. DE-OS 34 31 920; Chem. Ber. 90 (1957), 841 - 852; J. Org. Chem. 27 (1962), 1703 - 1709).

Die beim erfindungsgemäßen Verfahren zur Herstellung der neuen Sulfonyliso(thio)harnstoffe weiter als Ausgangsstoffe zu verwendenden Aminoverbindungen sind durch die Formel (III) allgemein definiert. In Formel (III) haben $R^3$, $R^4$ und Z vorzugsweise bzw. insbesondere die gleichen Bedeutungen, wie sie oben

im Rahmen der Substituentendefinition der Formel (I) vorzugsweise bzw. als insbesondere bevorzugt angegeben sind.

Beispiele für die Ausgangsstoffe der Formel (III) sind in der nachstehenden Tabelle 4 aufgeführt.

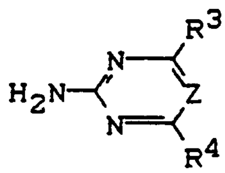

(III)

Tabelle 4: Beispiele für die Ausgangsstoffe der Formel (III)

| $R^3$ | $R^4$ | Z | $R^3$ | $R^4$ | Z |
|---|---|---|---|---|---|
| $CH_3$ | $CH_3$ | N | $CH_3$ | $OCH_3$ | N |
| $OCH_3$ | $OCH_3$ | N | $C_2H_5$ | $OCH_3$ | N |
| $CH_3$ | $OC_2H_5$ | N | $OC_2H_5$ | $OC_2H_5$ | N |

Die Verbindungen der Formel (III) sind bekannt und/oder können nach an sich bekannten Verfahren hergestellt werden (vgl. EP-A 121 082, EP-A 125 205, EP-A 126 711, EP-A 152 378, US-P 4 299 960).

Alkalimetall- bzw. Erdalkalimetall-salze hiervon können auf übliche Weise durch Umsetzung mit basischen Metallverbindungen, wie z. B. Natrium-, Kalium-, Magnesium-oder Calcium-hydrid sowie Natrium- oder Kalium-tert.-butylat, hergestellt werden.

Das erfindungsgemäße Verfahren zur Herstellung der neuen Sulfonyliso(thio)harnstoffe der Formel (I) wird unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran and Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Ether, wie z. B. Diethyl- und Dibutylether, Glykoldimethylether und Diglykoldimethylether, Tetrahydrofuran und Dioxan, werden als Verdünnungsmittel beim erfindungsgemäßen Verfahren ganz besonders bevorzugt verwendet.

Bei dem erfindungsgemäßen Verfahren können alle üblichen basischen Metallverbindungen eingesetzt werden. Vorzugsweise in Frage kommen Alkalimetallhydride, wie z. B. Lithium-, Natrium- und Kaliumhydrid, Erdalkalimetallhydride, wie z. B. Calciumhydrid, Alkalimetallhydroxide wie z. B. Natrium-und Kaliumhydroxid, Erdalkalihydroxide wie z. B. Calciumhydroxid, Alkalicarbonate und -alkoholate wie Natrium- und Kaliumcarbonat, Natrium- und Kalium-tert.-butylat.

Natrium und Kalium-tert.-butylat werden als basische Metallverbindungen beim erfindungsgemäßen Verfahren ganz besonders bevorzugt eingesetzt.

Die Reaktionstemperaturen können bei dem erfindungsgemäßen Verfahren in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren wird im allgemeinen unter Normaldruck durchgeführt. Es ist jedoch uch möglich, unter erhöhtem oder vermindertem Druck zu arbeiten.

Zur Durchführung des erfindungsgemäßen Verfahrens setzt man auf 1 Mol N-Sulfonyl-imino-(dithio)-kohlensäure-diester der Formel (II) im allgemeinen zwischen 0,5 und 2 Mol, vorzugsweise zwischen 0,8 und

1,2 Mol, Aminoverbindung der Formel (III) oder von deren Alkalimetall-bzw. Erdalkalimetall-salz ein.

Zur Durchführung des erfindungsgemäßen Verfahrens können die Reaktionskomponenten in beliebiger Reihenfolge vermischt und nach beendeter Umsetzung kann nach üblichen Methoden aufgearbeitet werden.

In einer bevorzugten Ausführungsform des erfindungsgemäßen Verfahrens wird zunächst die Aminoverbindung der Formel (III) mit einer geeigneten basischen Metallverbindung in einem Verdünnungsmittel umgesetzt. Dann wird der N-Sulfonyl-imino-(dithio)kohlensäure-diester der Formel (II) dazugegeben und das Reaktionsgemisch wird bis zum Ende der Umsetzung gerührt.

Zur Aufarbeitung wird mit Wasser verdünnt und mit einer starken Säure, wie z. B. Salzsäure, angesäuert, mit einem mit Wasser praktisch nicht mischbaren organischen Lösungsmittel, wie z. B. Methylenchlorid, extrahiert, die organische Phase mit Wasser gewaschen, getrocknet und anschließend unter vermindetem Druck eingeengt. Der Rückstand enthält im wesentlichen das Produkt der Formel (I). Er kann nach üblichen Methoden, beispielsweise durch Verreiben mit einem organischen Lösungsmittel, wie z. B. Ethanol, zur Kristallisation gebracht und durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen :

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur selektiven Bekämpfung von Unkräutern insbesondere in Weizen, Gerste und Reis, teilweise auch in Mais und Sojabohnen, vorzugsweise im Nachauflaufverfahren, eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion der N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen. Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

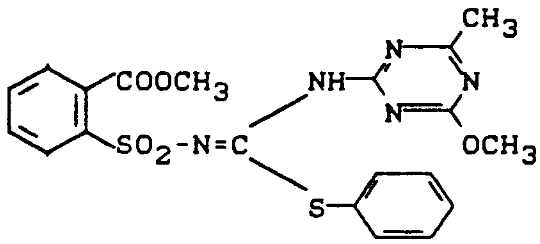

Eine Mischung aus 4,6 g (0,033 Mol) 2-Amino-4-methoxy-6-methyl-s-triazin, 2 g Natrium-hydrid und 150 ml Tetrahydrofuran wird 24 Stunden bei 20 °C gerührt. Dann werden 14,8 g (0,033 Mol) N-(2-Methoxycarbonyl-phenylsulfonyl)-imino-dithiokohlensäure-diphenylester portionsweise dazu gegeben und das Reaktionsgemisch wird 18 Stunden bei 20 °C gerührt. Nach Verdünnen mit 300 ml Wasser wird mit 2N-Salzsäure angesäuert und mit Methylenchlorid extrahiert. Die Methylenchlorid-Lösung wird mit Wasser gewaschen, mit Natriumsulfat getrocknet und filtriert. Vom Filtrat werden das Lösungsmittel und das Thiophenol unter vermindertem Druck abdestilliert. Der Rückstand wird mit Ethanol verrührt und das kristallin angefallene Produkt wird durch Absaugen isoliert.

Man erhält 11,0 g (71 % der Theorie) N′-(4-Methoxy-6-methyl-s-triazin-2-yl)-N′-(2-methoxycarbonyl-phenyl-sulfonyl)-S-phenyl-isothioharnstoff vom Schmelzpunkt 139 °C.

Analog Beispiel 1 und entsprechend der allgemeinen Beschreibung des erfindungsgemäßen Verfahrens können die in der nachstehenden Tabelle 5 aufgeführten Verbindungen der Formel (I) hergestellt werden.

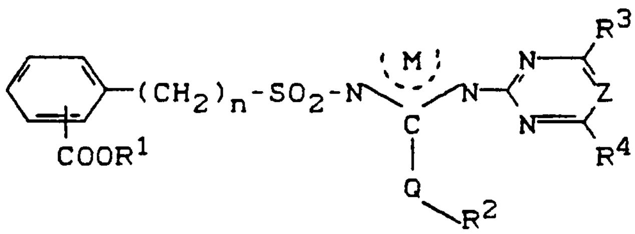

(I)

Tabelle 5: Beispiele für die Verbindungen der Formel (I)

Soweit nicht anders angegeben befindet sich die Gruppierung $COOR^1$ in der ortho-Position.

| Bsp.-Nr. | n | Q | $R^1$ | $R^2$ | $R^3$ | $R^4$ | M | Z | Schmelzpunkt (°C) |
|---|---|---|---|---|---|---|---|---|---|
| 2 | 0 | S | $CH_3$ | 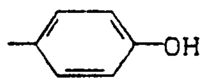 | $CH_3$ | $OCH_3$ | H | N | 116 |
| 3 | 0 | S | $CH_3$ | 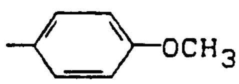 | $CH_3$ | $OCH_3$ | H | N | 134 |
| 4 | 0 | S | $CH_3$ | 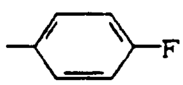 | $CH_3$ | $OCH_3$ | H | N | 123 |
| 5 | 0 | S | $CH_3$ | 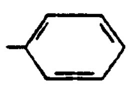 | $CH_3$ | $OCH_3$ | Na | N | |
| 6 | 0 | S | $CH_3$ | 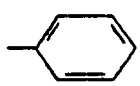 | $CH_3$ | $OCH_3$ | K | N | |

Tabelle 5 - Fortsetzung

| Bsp.-Nr. | n | Q | $R^1$ | $R^2$ | $R^3$ | $R^4$ | M | Z | Schmelz-punkt(°C) |
|---|---|---|---|---|---|---|---|---|---|
| 7 | 0 | S | $CH_3$ | 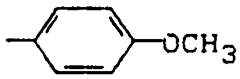 | $CH_3$ | $OCH_3$ | 1/2Ca | N | |
| 8 | 0 | S | $CH_3$ | 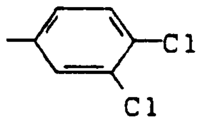 | $CH_3$ | $OCH_3$ | H | N | 146 |
| 9 | 0 | S | $CH_3$ | 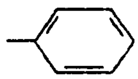 | $OCH_3$ | $OCH_3$ | H | N | |
| 10 | 0 | S | $CH_3$ | 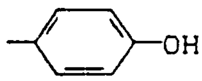 | $OCH_3$ | $OCH_3$ | H | N | |
| 11 | 0 | S | $CH_3$ | 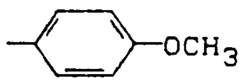 | $OCH_3$ | $OCH_3$ | H | N | |
| 12 | 0 | S | $CH_3$ | 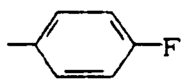 | $OCH_3$ | $OCH_3$ | H | N | |
| 13 | 0 | S | $CH_3$ | 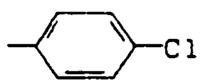 | $OCH_3$ | $OCH_3$ | H | N | |
| 14 | 0 | S | $CH_3$ | 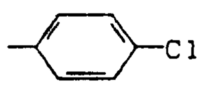 | $CH_3$ | $OCH_3$ | H | N | |
| 15 | 0 | S | $CH_3$ | 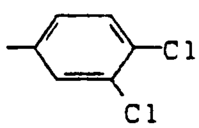 | $OCH_3$ | $OCH_3$ | H | N | |
| 16 | 0 | O | $CH_3$ | 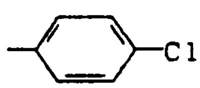 | $CH_3$ | $OCH_3$ | H | N | |

Tabelle 5 - Fortsetzung

| Bsp.-Nr. | n | Q | $R^1$ | $R^2$ | $R^3$ | $R^4$ | M | Z | Schmelzpunkt(°C) |
|---|---|---|---|---|---|---|---|---|---|
| 17 | 0 | O | $CH_3$ | 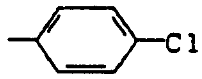 | $OCH_3$ | $OCH_3$ | H | N | |
| 18 | 0 | S | $C_2H_5$ | 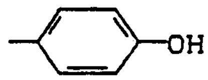 | $CH_3$ | $OCH_3$ | H | N | |
| 19 | 0 | S | $C_2H_5$ | 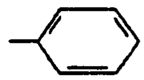 | $OCH_3$ | $OCH_3$ | H | N | |

Tabelle 5 - Fortsetzung

| Bsp.-Nr. | n | Q | $R^1$ | $R^2$ | $R^3$ | $R^4$ | M | Z | Schmelz-punkt(°C) |
|---|---|---|---|---|---|---|---|---|---|
| 20 | 0 | S | $C_2H_5$ | 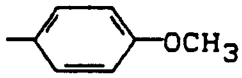 | $OCH_3$ | $OCH_3$ | H | N | |
| 21 | 0 | S | $C_2H_5$ | 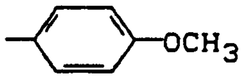 | $CH_3$ | $OCH_3$ | H | N | |
| 22 | 0 | S | $C_2H_5$ | 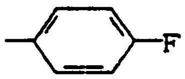 | $CH_3$ | $OCH_3$ | H | N | |
| 23 | 0 | S | $C_2H_5$ | 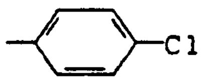 | $OCH_3$ | $OCH_3$ | H | N | |
| 24 | 0 | S | $C_2H_5$ | 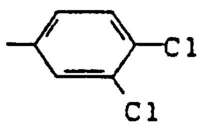 | $OCH_3$ | $OCH_3$ | H | N | |
| 25 | 0 | S | $C_2H_5$ | 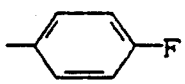 | $OCH_3$ | $OCH_3$ | H | N | |
| 26 | 0 | S | $C_2H_5$ | 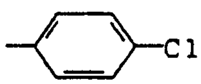 | $CH_3$ | $OCH_3$ | H | N | |
| 27 | 0 | S | $C_2H_5$ | 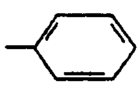 | $CH_3$ | $OCH_3$ | H | N | |

Tabelle 5 - Fortsetzung

| Bsp.-Nr. | n | Q | $R^1$ | $R^2$ | $R^3$ | $R^4$ | M | Z | Schmelzpunkt (°C) |
|---|---|---|---|---|---|---|---|---|---|
| 28 | 0 | O | $CH_3$ | 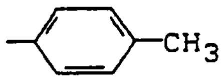 | $CH_3$ | $OCH_3$ | H | N | |
| 29 | 0 | O | $CH_3$ | 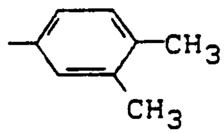 | $CH_3$ | $OCH_3$ | H | N | |
| 30 | 0 | O | $CH_3$ | 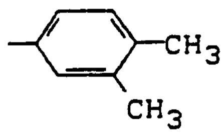 | $OCH_3$ | $OCH_3$ | H | N | |
| 31 | 0 | O | $CH_3$ | 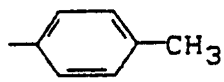 | $OCH_3$ | $OCH_3$ | H | N | |
| 32 | 0 | O | $CH_3$ | 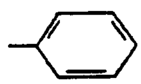 | $OCH_3$ | $OCH_3$ | H | N | |
| 33 | 0 | O | $CH_3$ | 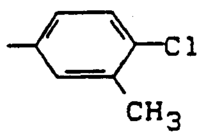 | $OCH_3$ | $OCH_3$ | H | N | |

Tabelle 5 - Fortsetzung

| Bsp.-Nr. | n | Q | $R^1$ | $R^2$ | $R^3$ | $R^4$ | M | Z | Schmelzpunkt(°C) |
|---|---|---|---|---|---|---|---|---|---|
| 34 | 0 | O | $CH_3$ | 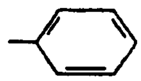 | $CH_3$ | $OCH_3$ | H | N | |
| 35 | 0 | S | $CH_3$ | 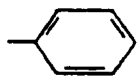 | $CH_3$ | $OC_2H_5$ | H | N | |
| 36 | 0 | O | $CH_3$ | 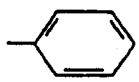 | $CH_3$ | $OC_2H_5$ | H | N | |
| 37 | 0 | S | $CH_3$ | 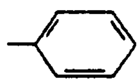 | $C_2H_5$ | $OCH_3$ | H | N | |
| 38 | 0 | O | $CH_3$ | 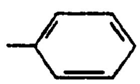 | $C_2H_5$ | $OCH_3$ | H | N | |
| 39 | 0 | O | $C_2H_5$ | 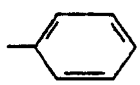 | $CH_3$ | $OCH_3$ | H | N | |
| 40 | 0 | O | $C_2H_5$ | 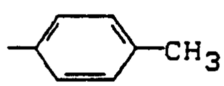 | $CH_3$ | $OCH_3$ | H | N | |
| 41 | 0 | O | $C_2H_5$ | 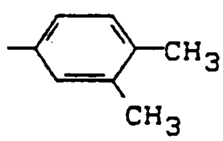 | $CH_3$ | $OCH_3$ | H | N | |
| 42 | 0 | O | $C_2H_5$ | 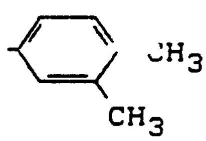 | $OCH_3$ | $OCH_3$ | H | N | |

Ausgangsstoffe der Formel (II)

Beispiel (II-1)

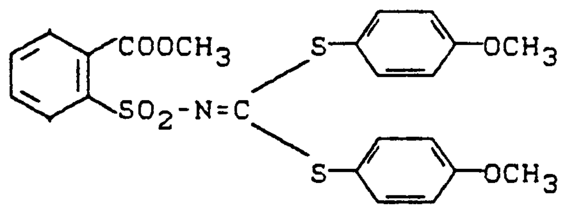

Eine Lösung von 7,4 g (0,05 Mol) N-(2-Methoxy-carbonyl-phenylsulfonyl)-isocyanid-dichlorid in 75 ml Toluol wird bei 22 °C unter Rühren zu einer Mischung aus 14,0 g (0,10 Mol) 4-Methoxy-thiophenol, 10,0 g (0,10 Mol) Triethylamin und 100 ml Toluol tropfenweise gegeben. Das Reaktionsgemisch wird 18 Stunden bei 22 °C gerührt und dann mit 200 ml Wasser geschüttelt. Die organische Phase wird abgetrennt, mit Natriumsulfat getrocknet und filtriert. Das Filtrat wird eingeengt, der Rückstand mit Ethanol verrieben und das kristallin angefallene Produkt durch Absaugen isoliert.

Man erhält 16,6 g (66 % der Theorie) N-(2-Methoxycarbonyl-phenylsulfonyl)-imino-dithiokohlensäure-S,S-bis-(4-methoxy-phenyl)-ester vom Schmelzpunkt 101 °C.

Analog Beispiel (II-1) können die in der nachstehenden Tabelle 6 aufgeführten Verbindungen der Formel (II) - wie auch die in Tabelle 1 aufgeführten Verbindungen - hergestellt werden.

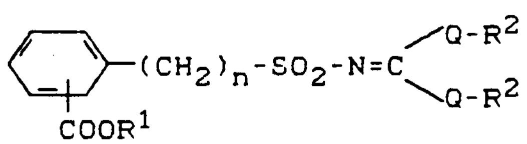

(II)

Tabelle 6: Beispiele für die Verbindungen der Formel (II)

| Beip.-Nr. | n | Q | $R^1$ | $R^2$ | Position $COOR^1$ | Schmelzpunkt (°C) |
|---|---|---|---|---|---|---|
| II-2 | 0 | S | $CH_3$ | 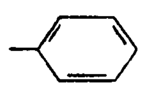 | ortho | 91 |
| II-3 | 0 | S | $CH_3$ | 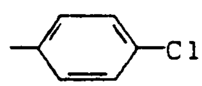 | ortho | 139 |
| II-4 | 0 | S | $CH_3$ | 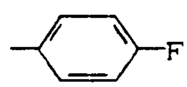 | ortho | 135 |

Ausgangsstoffe der Formel (IV)

Beispiel (IV-1)

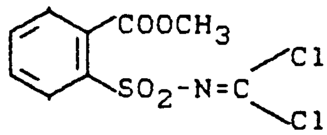

135 g (1 Mol) Sulfurylchlorid werden tropfenweise so zu einer Mischung aus 27 g (0,085 Mol) N-(2-Methoxycarbonyl-phenylsulfonyl)-imino-dithiokohlensäure-dimethylester und 200 ml Chloroform gegeben, daß die Innentemperatur 55 °C nicht übersteigt. Das komplette Reaktionsgemisch wird noch eine Stunde bei 60 °C gerührt und das Reaktionsprodukt wird anschließend durch fraktionierte Destillation isoliert.

Man erhält 22 g (91 % der Theorie) N-(2-Methoxycarbonyl-phenylsulfonyl)-isocyanid-dichlorid vom Siedepunkt 184 °C bei 0,3 kPa.

Analog Beispiel (IV-1) können die oben in Tabelle 2 aufgeführten Verbindungen der Formel (IV) hergestellt werden.

Ausgangsstoffe der Formel (VI)

Beispiel (VI-1)

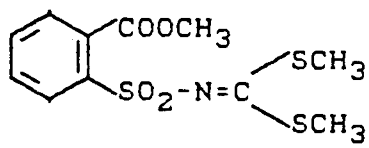

Zu 7,4 g (0,05 Mol) Iminodithiokohlensäuredimethylester in 100 ml Methylenchlorid werden bei 0 °C zuerst 11,2 g (0,1 Mol) 1,4-Diazabicyclo-[2.2.2 ]-octan - gelöst in 50 ml Methylenchlorid - und dann 11,7 g (0,05 Mol) 2-Methoxycarbonylbenzolsulfonsäurechlorid - gelöst in 50 ml Methylenchorid - getropft. Man läßt dann die Temperatur auf 20 - 25 °C steigen und rührt 18 Stunden nach. Das Reaktionsgemisch wird dann einmal mit verdünnter Salzsäure und zweimal mit Wasser gewaschen. Nach dem Eindampfen der Methylenchloridphase wird der Rückstand mit Alkohol verrieben.

Man erhält 12,5 g (78,3 % der Theorie N-(2-Methoxycarbonyl-phenylsulfonyl)-imino-dithiokohlensäure-dimethylester in Form farbloser Kristalle vom Schmelzpunkt 96 °C.

Analog Beispiel (VI-1) können die oben in Tabelle 3 aufgeführten Verbindungen der Formel (VI) hergestellt werden.

Anwendungsbeispiele

In den folgenden Anwendungsbeispielen wird die nachstehend aufgeführte Verbindung als Vergleichssubstanz herangezogen:

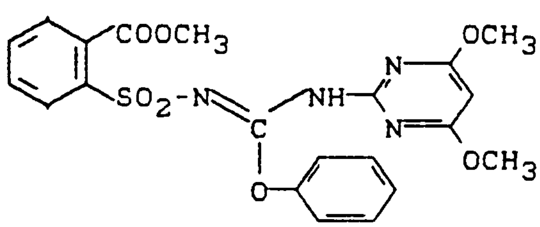

(A)

N′-(2-Methoxycarbonyl-phenylsulfonyl)-N″-(4,6-dimethoxy-pyrimidin-2-yl)-O-phenyl-isoharnstoff (bekannt aus EP-A 173 957).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden aufgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Beispielsweise zeigt in diesem Test die Verbindung gemäß Herstellungsbeispiel (2) im Vergleich mit der bekannten Verbindung (A) bessere Verträglichkeit gegenüber Kulturpflanzen, wie z. B. Weizen, und stärkere Wirkung gegen Unkräuter, wie z. B. Chenopodium, Abutilon, Ipomoea und Portulaca.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Verbindungen zeigen in diesem Test eine sehr gute herbizide Wirksamkeit. Beispielsweise zeigen in diesem Test die Verbindungen gemäß den Herstellungsbeispielen (2), (3), (4) und (8) im Vergleich mit der bekannten Verbindung (A) bessere Verträglichkeit gegenüber Kulturpflanzen, wie z. B. Weizen, und stärkere Wirkung gegen Unkräuter, wie z. B. Chenopodium, Ipomoea, Portulaca, Veronica und Viola.

**Patentansprüche**

**1.** Sulfonyliso(thio)harnstoffe der allgemeinen Formel (I)

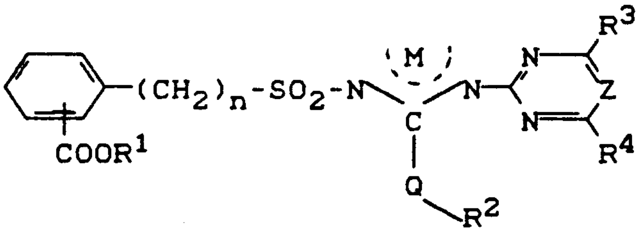

(I)

in welcher

M für Wasserstoff oder ein Alkalimetall- oder Erdalkalimetall-äquivalent steht,

n für die Zahlen Null oder 1 steht,

Q für Sauerstoff oder Schwefel steht,

$R^1$ für gegebenenfalls durch Halogen oder $C_1$-$C_4$-Alkoxy substituiertes $C_1$-$C_6$-Alkyl steht,

$R^2$ für einen Phenylrest steht, welcher gegebenenfalls substituiert ist durch einen oder mehrere Reste aus der Reihe
Halogen [wie insbesondere Fluor, Chlor, Brom und Iod], Cyano, Nitro, Hydroxy, Carboxy, $C_1$-$C_6$-Alkyl[welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkylamino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonylamino, $C_1$-$C_4$-Alkoxy-carbonylamino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkylamino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist;

$R^3$ für gegebenenfalls durch Halogen substituiertes $C_1$-$C_2$-Alkyl oder $C_1$-$C_2$-Alkoxy steht,

$R^4$ für gegebenenfalls durch Halogen substituiertes $C_1$-$C_2$-Alkyl oder $C_1$-$C_2$-Alkoxy steht und

Z für N steht.

**2.** Verbindungen der Formel (I) gemäß Anspruch 1, dadurch gekennzeichnet, daß darin

M für Wasserstoff oder ein Natrium-, Kalium-, Magnesium- oder Calcium-äquivalent steht,

n für die Zahlen Null oder 1 steht,

Q für Sauerstoff oder Schwefel steht,

$R^1$ für Methyl, Ethyl, Propyl, Isopropyl, Butyl, Isobutyl, sec-Butyl, 2-Fluor-ethyl, 2-Chlor-ethyl, 2-Methoxy-ethyl oder 2-Ethoxyethyl steht,

$R^2$ für einen Phenylrest steht, welcher gegebenenfalls substituiert ist durch einen oder zwei Reste aus der Reihe
Fluor, Chlor, Brom, Iod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pydridoxy [welches gegebenenfalls durch Chlor und/oder Trifluormethyl substituiert ist], oder welcher gegebenenfalls benzanelliert ist;

$R^3$ für Methyl, Ethyl, Trifluormethyl, Methoxy, Ethoxy, Difluormethoxy oder Trifluorethoxy steht,

$R^4$ für Methyl, Methoxy, Ethoxy, Difluormethoxy oder Trifluorethoxy steht und

Z für N steht.

**3.** Verbindungen der Formel (I) gemäß Anspruch 1, dadurch gekennzeichnet, daß darin
- M für Wasserstoff steht,
- n für Null steht,
- Q für Schwefel steht,
- $R^1$ für Methyl oder Ethyl steht,
- $R^2$ für gegebenenfalls durch Fluor, Chlor, Methyl oder Methoxy substituiertes Phenyl steht,
- $R^3$ für Methyl oder Methoxy steht,
- $R^4$ für Methoxy steht und
- Z für N steht
- mit der Maßgabe, daß sich die Gruppierung $COOR^1$ in der ortho-Position befindet.

**4.** Verfahren zur Herstellung der Sulfonyliso(thio)harnstoffe der allgemeinen Formel (I) gemäß Anspruch 1, dadurch gekennzeichnet, daß man N-Sulfonyl-imino-(dithio)kohlensäure-diester der allgemeinen Formel (II)

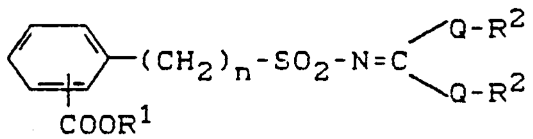
(II)

in welcher
n, Q, $R^1$ und $R^2$ die in Anspruch 1 angegebenen Bedeutungen haben,
mit Aminoverbindungen der allgemeinen Formel (III)

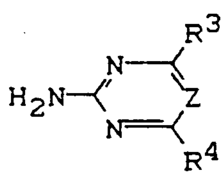
(III)

in welcher
$R^3$, $R^4$ und Z die in Anspruch 1 angegebenen Bedeutungen haben,
oder mit Alkalimetall- bzw. Erdalkalimetall-salzen hiervon in Gegenwart eines Verdünnungsmittels und gegebenenfalls in Gegenwart einer basischen Metallverbindung bei Temperaturen zwischen 0 °C und 100 °C umsetzt.

**5.** N-Sulfonyl-imino-(dithio)kohlensäure-diester der allgemeinen Formel

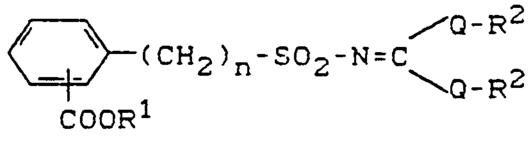
(II)

in welcher
n, Q, $R^1$ und $R^2$ die in Anspruch 1 angegebenen Bedeutungen haben.

**6.** Sulfonyl-isocyanid-dichlorlde der allgemeinen Formel

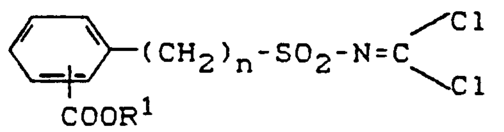

(IV)

in welcher

n und $R^1$ die in Anspruch 1 angegebenen Bedeutungen haben.

**7.** Herbizide Mittel, gekennzeichnet durch einen Gehalt an mindestens einem Sulfonyliso(thio)harnstoff der allgemeinen Formel (I) gemäß Anspruch 1.

**8.** Verwendung von Sulfonyliso(thio)harnstoffen der allgemeinen Formel (I) gemäß Anspruch 1 zur Bekämpfung von unerwünschtem Pflanzenwachstum.

**9.** Verfahren zur Herstellung von herbiziden Mitteln, dadurch gekennzeichnet, daß man Sulfonyliso(thio)-harnstoffe der allgemeinen Formel (I) gemäß Anspruch 1 mit Streckmitteln und/oder oberflächenaktiven Mitteln vermischt.

## Claims

**1.** Sulphonyliso(thio)ureas of the general formula (I)

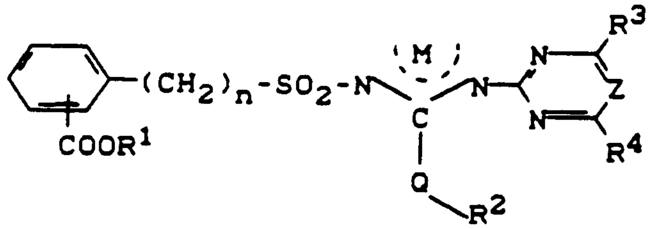

(I)

in which

M represents hydrogen or an alkali metal equivalent or an alkaline earth metal equivalent,

n represents the numbers zero or 1,

Q represents oxygen or sulphur,

$R^1$ represents $C_1$-$C_6$-alkyl which is optionally substituted by substituents from the series comprising halogen and $C_1$-$C_4$-alkoxy,

$R^2$ represents a phenyl radical, which is optionally substituted by one or more radicals from the series comprising

halogen [such as fluorine, chlorine, bromine and iodine in particular], cyano, nitro, hydroxyl, carboxyl, $C_1$-$C_6$-alkyl [which is optionally substituted by fluorine, chlorine, bromine, nitro, cyano, hydroxyl, carboxyl, $C_1$-$C_4$-alkoxy-carbonyl, $C_1$-$C_4$-alkoxy, $C_1$-$C_4$-alkylthio or phenyl], $C_3$-$C_6$-cycloalkyl, $C_1$-$C_4$-alkoxy [which is optionally substituted by fluorine, chlorine, bromine, cyano, carboxyl, $C_1$-$C_4$-alkoxy, $C_1$-$C_4$-alkylthio or $C_1$-$C_4$-alkoxy-carbonyl], $C_1$-$C_4$-alkylthio [which is optionally substituted by fluorine, chlorine, bromine, cyano, carboxyl, $C_1$-$C_4$-alkoxy-carbonyl], amino, $C_1$-$C_4$-alkylamino or di-($C_1$-$C_4$-alkyl)-amino [which are optionally substituted by fluorine, chlorine, bromine, cyano, carboxyl, $C_1$-$C_4$-alkoxy or $C_1$-$C_4$-alkoxy-carbonyl], $C_1$-$C_4$-alkyl-carbonylamino, $C_1$-$C_4$-alkoxy-carbonylamino, (di)-$C_1$-$C_4$-alkylamino-carbonylamino, formyl, $C_1$-$C_4$-alkyl-carbonyl, benzoyl, C,-$C_4$-alkoxy-carbonyl, phenoxy-carbonyl, benzyloxycarbonyl, phenyl [which is optionally substituted by fluorine, chlorine, bromine, cyano, nitro, hydroxyl or methyl], phenoxy, phenylthio, phenylsulphonyl, phenylamino or phenylazo [which are optionally substituted by fluorine, chlorine, bromine, cyano, nitro, methyl and/or trifluoromethyl], pyridoxy or pyrimidoxy [which are optionally substituted by fluorine, chlorine, bromine, cyano, nitro, methyl and/or trifluoromethyl], $C_1$-$C_4$-alkyl-carbonyloxy, $C_1$-$C_4$-alkoxy-carbonyloxy, $C_1$-$C_4$-alkyl-amino-carbonyloxy and di-($C_1$-$C_4$-alkyl)-amino-carbonyloxy, or which is optionally anellated by an alkylene chain [which is optionally branched and/or interrupted

by one or more oxygen atoms] or a benzene radical [which is optionally substituted by fluorine, chlorine, bromine, cyano, nitro, methyl and/or trifluoromethyl];

$R^3$ represents $C_1$-$C_2$-alkyl or $C_1$-$C_2$-alkoxy which are optionally substituted by halogen,

$R^4$ represents $C_1$-$C_2$-alkyl or $C_1$-$C_2$-alkoxy which are optionally substituted by halogen, and

Z represents N.

**2.** Compounds of the formula (I) according to Claim 1, characterized in that therein

M represents hydrogen or a sodium, potassium, magnesium or calcium equivalent,

n represents the numbers zero or 1,

Q represents oxygen or sulphur,

$R^1$ represents methyl, ethyl, propyl, isopropyl, butyl, isobutyl, sec.-butyl, 2-fluoro-ethyl, 2-chloro-ethyl, 2-methoxy-ethyl or 2-ethoxy-ethyl,

$R^2$ represents a phenyl radical, which is optionally substituted by one or two radicals from the series comprising fluorine, chlorine, bromine, iodine, cyano, nitro, hydroxyl, carboxyl, $C_1$-$C_3$-alkoxy-carbonyl, $C_1$-$C_4$-alkyl, trifluoromethyl, hydroxymethyl, methoxy-carbonylmethyl, phenyl-$C_1$-$C_3$-alkyl, cyclohexyl, $C_1$-$C_3$-alkoxy, trifluoromethoxy, $C_1$-$C_3$-alkylthio, trifluoromethylthio, dimethylamino, amino, acetamido, methylaminocarbonyl, formyl, acetyl, benzoyl, phenyl, hydroxyphenyl, phenoxy [which is optionally substituted by chlorine and/or trifluoromethyl], phenylamino, phenylazo, pyridoxy [which is optionally substituted by chlorine and/or trifluoromethyl], or which is optionally benzanellated;

$R^3$ represents methyl, ethyl, trifluoromethyl, methoxy, ethoxy, difluoromethoxy or trifluoroethoxy,

$R^4$ represents methyl, methoxy, ethoxy, difluoromethoxy or trifluoromethoxy, and

Z represents N.

**3.** Compounds of the formula (I) according to Claim 1, characterized in that therein

M represents hydrogen,

n represents zero,

Q represents sulphur,

$R^1$ represents methyl or ethyl,

$R^2$ represents phenyl which is optionally substituted by a substituent from the series comprising fluorine, chlorine, methyl and methoxy,

$R^3$ represents methyl or methoxy,

$R^4$ represents methoxy, and

Z represents N,

- with the proviso that the $COOR^1$ group is situated in the ortho-position.

**4.** Process for the preparation of the sulphonyliso(thio)ureas of the general formula (I) according to Claim 1, characterized in that N-sulphonyl-imino-(dithio)carbonic acid diesters of the general formula (II)

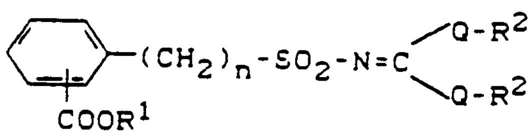

(II)

in which

n, Q, $R^1$ and $R^2$ have the meanings given in Claim 1, are reacted with amino compounds of the general formula (III)

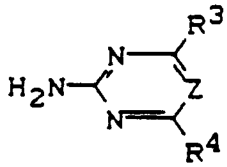

(III)

in which
$R^3$, $R^4$ and Z have the meanings given in Claim 1, or with alkali metal salts or alkaline earth metal salts thereof in the presence of a diluent and if appropriate in the presence of a basic metal compound, at temperatures between 0 ° C and 100 ° C.

**5.** N-Sulphonyl-imino-(dithio)carbonic acid diesters of the general formula

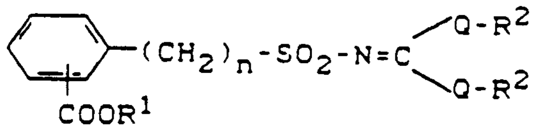

(II)

in which
n, Q, $R^1$ and $R^2$ have the meanings given in Claim 1.

**6.** Sulphonylisocyanide dichlorides of the general formula

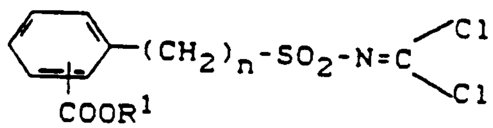

(IV)

in which
n and $R^1$ have the meanings given in Claim 1.

**7.** Herbicidal agents, characterized in that they contain at least one sulphonyliso(thio)urea of the general formula (I) according to Claim 1.

**8.** Use of sulphonyliso(thio)ureas of the general formula (I) according to Claim 1 for combating undesired plant growth.

**9.** Process for the production of herbicidal agents, characterized in that sulphonyliso(thio)ureas of the general formula (I) according to Claim 1 are mixed with extenders and/or surface-active agents.

**Revendications**

**1.** Sulfonyliso(thio)urées de formule générale (I)

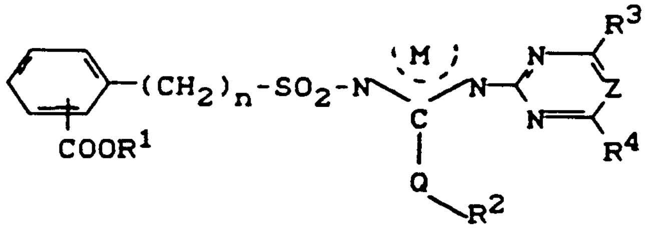

(I)

dans laquelle

M représente l'hydrogène ou un équivalent de métal alcalin ou de métal alcalino-terreux,
n représente le nombre zéro ou 1,
Q représente l'oxygène ou le soufre,
$R^1$ est un groupe alkyle en $C_1$ à $C_6$ éventuellement substitué par un halogène ou un radical

alkoxy en $C_1$ à $C_4$,

$R^2$ représente un reste phényle qui est éventuellement substitué par un ou plusieurs restes de la série
halogène [tel que notamment fluor, chlore, brome et iode], cyano, nitro, hydroxy, carboxy, alkyle en $C_1$ à $C_6$ [qui porte éventuellement un substituant fluoro, chloro, bromo, nitro, cyano, hydroxy, carboxy, (alkoxy en $C_1$ à $C_4$)-carbonyle, alkoxy en $C_1$ à $C_4$, alkylthio en $C_1$ à $C_4$ ou phényle], cycloalkyle en $C_3$ à $C_6$, alkoxy en $C_1$ à $C_4$ [qui est éventuellement substitué par un radical fluoro, chloro, bromo, cyano, carboxy, alkoxy en $C_1$ à $C_4$, alkylthio en $C_1$ à $C_4$ ou (alkoxy en $C_1$ à $C_4$)-carbonyle], alkylthio en $C_1$ à $C_4$ [qui porte éventuellement un substituant fluoro, chloro, bromo, cyano, carboxy, (alkoxy en $C_1$ à $C_4$)-carbonyle], amino, alkylamino en $C_1$ à $C_4$ ou di-(alkyle en $C_1$ à $C_4$)-amino [qui portent le cas échéant un substituant fluoro, chloro, bromo, cyano, carboxy, alkoxy en $C_1$ à $C_4$ ou (alkoxy en $C_1$ à $C_4$)-carbonyle], (alkyle en $C_1$ à $C_4$)-carbonylamino, (alkoxy en $C_1$ à $C_4$)-carbonylamino, (di)-(alkyle en $C_1$ à $C_4$)-amino-carbonyl-amino, formyle, (alkyle en $C_1$ à $C_4$)-carbonyle, benzoyle, (alkoxy en $C_1$ à $C_4$)-carbonyle, phénoxy-carbonyle, benzyloxy-carbonyle, phényle [qui porte le cas échéant un substituant fluoro, chloro, bromo, cyano, nitro, hydroxy ou méthyle], phénoxy, phénylthio, phénylsulfonyle, phénylamino ou phénylazo (qui portent le cas échéant un substituant fluoro, chloro, bromo, cyano, nitro, méthyle et/ou trifluorométhyle], pyridoxy ou pyrimidoxy [qui portent le cas échéant un substituant fluoro, chloro, bromo, cyano, nitro, méthyle et/ou trifluorométhyle], (alkyle en $C_1$ à $C_4$)-carbonyloxy, (alkoxy en $C_1$ à $C_4$)-carbonyloxy, (alkyle en $C_1$ à $C_4$)-amino-carbonyloxy et di-(alkyle en $C_1$ à $C_4$)-amino-carbonyloxy, ou qui est condensé le cas échéant par une chaine alkylène [qui est éventuellement ramifiée et/ou interrompue par un ou plusieurs atomes d'oxygène] ou un reste benzo [qui est substitué le cas échéant par du fluor, du chlore, du brome ou un radical cyano, nitro, méthyle et/ou trifluorométhyle] ;

$R^3$ est un groupe alkyle en $C_1$ ou $C_2$ ou alkoxy en $C_1$ ou $C_2$ portant éventuellement un substituant halogéno,

$R^4$ est un groupe alkyle en $C_1$ ou $C_2$ ou un groupe alkoxy en $C_1$ ou $C_2$ portant éventuellement un substituant halogéno et

Z représente N.

**2.** Composés de formule (I) suivant la revendication 1, caractérisés en ce que dans cette formule

M représente l'hydrogène ou un équivalent de sodium, de potassium, de magnésium ou de calcium,

n représente le nombre 0 ou 1,

Q est l'oxygène ou le soufre,

$R^1$ est un groupe méthyle, éthyle, propyle, isopropyle, butyle, isobutyle, sec-butyle, 2-fluoréthyle, 2-chloréthyle, 2-méthoxyéthyle ou 2-éthoxyéthyle,

$R^2$ représente un reste phényle qui est éventuellement substitué par un ou deux restes de la série fluor, chlore, brome, iode, cyano, nitro, hydroxy, carboxy, (alkoxy en $C_1$ à $C_3$)-carbonyle, alkyle en $C_1$ à $C_4$, trifluorométhyle, hydroxyméthyle, méthoxycarbonylméthyle, phényl-(alkyle en $C_1$ à $C_3$, cyclohexyle, alkoxy en $C_1$ à $C_3$, trifluorométhoxy, alkylthio en $C_1$ à $C_3$, trifluorométhylthio, diméthylamino, amino, acétylamino, méthylamino-carbonyle, formyle, acétyle, benzoyle, phényle, hydroxyphényle, phénoxy [qui est substitué le cas échéant par du chlore et/ou un radical trifluorométhyle], phénylamino, phénylazo, pyridoxy [qui est substitué le cas échéant par du chlore et/ou un radical trifluorométhyle], ou qui est éventuellement condensé au benzène ;

$R^3$ est un groupe méthyle, éthyle, trifluorométhyle, méthoxy, éthoxy, difluorométhoxy ou trifluoréthoxy,

$R^4$ est un groupe méthyle, méthoxy, éthoxy, difluorométhoxy ou trifluoréthoxy et

Z représente N.

**3.** Composés de formule (I) suivant la revendication 1, caractérisés en ce que dans cette formule

M représente l'hydrogène,

n est égal à zéro,

Q représente le soufre,

$R^1$ est un groupe méthyle ou éthyle,

$R^2$ est un groupe phényle éventuellement substitué par du fluor, du chlore ou un radical méthyle

ou méthoxy,
$R^3$ est un groupe méthyle ou méthoxy,
$R^4$ est un groupe méthoxy et
Z représente M
- sous réserve que le groupement $COOR^1$ se trouve en position ortho.

**4.** Procédé de production des sulfonyliso(thio)urées de formule générale (I) suivant la revendication 1, caractérisé en ce qu'on fait réagir des diesters d'acide N-sulfonyl-imino(dithio)carbonique de formule générale (II)

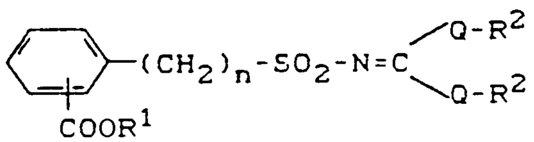

(II)

dans laquelle
n, Q, $R^1$ et $R^2$ ont les définitions données dans la revendication 1,
avec des composés aminés de formule générale (III)

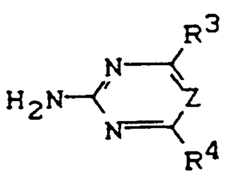

(III)

dans laquelle
$R^3$, $R^4$ et Z ont les définitions indiquées dans la revendication 1,
ou avec des sels de métaux alcalins ou de métaux alcalino-terreux de ces composés en présence d'un diluant et, le cas échéant, en présence d'un composé métallique basique à des températures comprises entre 0°C et 100°C.

**5.** Diesters d'acide N-sulfonyl-imino(dithio)carbonique de formule générale

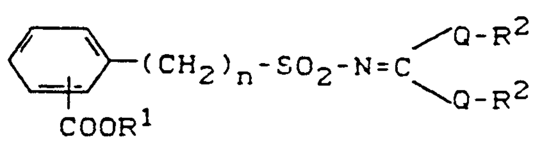

(II)

dans laquelle
n, Q, $R^1$ et $R^2$ ont les définitions indiquées dans la revendication 1.

**6.** Dichlorures d'isocyanure de sulfonyle de formule générale

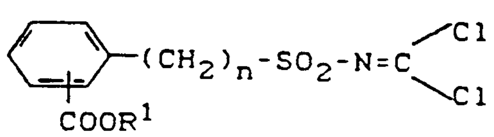

(IV)

dans laquelle
n et $R^1$ ont les définitions indiquées dans la revendication 1.

7. Compositions herbicides, caractérisées par une teneur en au moins une sulfonyliso(thio)urée de formule générale (I) suivant la revendication 1.

8. Utilisation de sulfonyliso(thio)urées de formule générale (I) suivant la revendication 1 pour combattre une croissance végétale non désirée.

9. Procédé de préparation de compositions herbicides, caractérisé en ce qu'on mélange des sulfonyliso-(thio)urées de formule générale (I) suivant la revendication 1 avec des diluants et/ou des agents tensio-actifs.